# EUROPÄISCHE PATENTSCHRIFT

(11) **EP 1 749 883 B1**
(45) Veröffentlichungstag und Bekanntmachung des Hinweises auf die Patenterteilung: **03.02.2010**
(21) Anmeldenummer: 06015060.4
(22) Anmeldetag: 19.07.2006
(51) Int. Cl.: C12M 1/107, C12M 1/18

(54) **Biogasanlage und Modul für eine Biogasanlage**
Biogas plant and module for a biogas plant
Installation de biogaz et module pour une Installation de biogaz

(30) Priorität: 05.08.2005 DE 202005012340 U
(43) Veröffentlichungstag der Anmeldung: 07.02.2007
(73) Patentinhaber: agraferm technologies AG, 85276 Pfaffenhofen/Ilm (DE)
(72) Erfinder: Friedmann, Johann, Dr., 85298 Scheyern (DE); Heck, Christian, Bissen (LU)
(74) Vertreter: Ganahl, Bernhard

(56) Entgegenhaltungen:
- EP-A2- 0 264 567
- WO-A-94/19120
- DE-A1- 19 532 359
- DE-A1- 19 744 653
- DE-A1- 19 958 142
- US-A- 5 656 491
- BAYERISCHES LANDESAMT FÜR UMWELTSCHUTZ: "Biogashandbuch Materialienband" BIOGASHANDBUCH BAYERN, [Online] Dezember 2004 (2004-12), XP002407797 Augsburg Gefunden im Internet: URL:http://www.bayern.de/lfu/abfall/biogas handbuch/> [gefunden am 2006-11-15]

## Beschreibung

Die Erfindung betrifft eine Biogasanlage nach dem Oberbegriff des Anspruchs 1 und ein Modul für eine Biogasanlage nach dem Oberbegriff des Anspruchs 7.

Biogas ist ein durch den anaeroben, mikrobiellen Abbau von organischen Stoffen entstehendes Gasgemisch, das zu 50 bis 70 % aus dem hochwertigen Energieträger Methan (CH₄) besteht. Weitere Bestandteile sind 30 bis 40 % Kohlendioxid (CO₂) sowie Spuren von Schwefelwasserstoff, Stickstoff, Wasserstoff und Kohlenmonooxid.

Aufgrund des relativ hohen Energiegehaltes lässt sich Biogas als Energieträger für die Wärme- und Krafterzeugung nutzen. Der durchschnittliche Heizwert von Biogas beträgt etwa 6000 kcal/m³ (= 25000 kJ/m³). Der Heizwert eines m³ Biogas entspricht demzufolge etwa 0,6 I Heizöl.

Zur Erzeugung des Biogases werden Gärbehälter verwendet, wobei das entstehende Biogas in Kesseln oder Motoren zur Stromerzeugung verbrannt wird. Als Ausgangsstoffe für die Biogaserzeugung kommen grundsätzlich alle Arten von Biomasse in Frage, deren Hauptkomponenten Kohlenhydrate, Eiweiße, Fette sowie Cellulose sind. Die für die Biogasproduktion nutzbaren organischen Stoffe sind zumeist Rest-oder Nebenprodukte verschiedener Branchen und Bereiche. Beispielsweise werden aus der Landwirtschaft Flüssig- und Festmist, Reststoffe der Pflanzenproduktion, aber auch extra hierfür angebaute Pflanzen wie beispielsweise Mais verwendet. Ferner können pflanzliche Rückstände der Bauereien und der Gemüse verarbeitenden Industrie sowie organische Schlämme und Abwässer der industriellen Aufarbeitung verwendet werden. Darüber hinaus ist es auch möglich tierische Erzeugnisse oder Erzeugnisse der Kommunalentsorgung zu verwenden. Grundsätzlich besitzen Biogasanlagen einen Fermenter zum Vergären des Substrates. Bekannt ist, Fermenter einzusetzen, die beispielsweise ein Volumen von 150 bis 1000 m³ haben. Die Fermenter können im Einzelfall auch wesentlich größer sein. So ist ein Fermenter für eine Biogasanlage mit einem Volumen von 8000 m³ bekannt. Im Fermenter verweilt das Substrat mehrere Tage, wobei durch die Aktivität der Mikroorganismen Biogas gebildet wird. Durch biochemische Umwandlung wird im Fermenter das Biogas entschwefelt, wobei Schwefelwasserstoff in elementaren Schwefel umgewandelt wird. Um die Bildung von Schwimmdecken und Sinkschichten zu verhindern wird das Substrat je nach Zusammensetzung gerührt. Hierdurch wird zusätzlich das Entweichen der entstehenden Gase erleichtert. Das vergorene Substrat wird anschließend in ein Endlager abgeführt, das möglichst geschlossen sein sollte, da Restbiogas ausgasen kann.

Das frisch erzeugte Biogas wird anschließend getrocknet und mit einem Sicherheitsfilter gereinigt.

Die aus der Vergärung übrig gebliebene Biomasse eignet sich als biologischer Dünger.

Eine sehr effiziente Nutzung der Energiequelle Biogas stellt der Betrieb eine Kraft-Wärme-Kopplung in einem Blockheizkraftwerk dar. Das Biogas dient dabei als Brennstoff für einen Diesel- oder Benzinmotor, der zur Stromgewinnung einen Generator antreibt. Die anfallende Abwärme des Motors kann als Heizwärme für den Fermenter genutzt werden, wobei der produzierte Strom in das Stromnetz eingespeist wird.

Es ist insbesondere bekannt, die Bestandteile einer Biogasanlage modular aufzubauen, d. h. beispielsweise die Blockheizkraftwerke als Module zu verwenden. Diese Module sind üblicherweise in an sich bekannten Containern untergebracht, wobei entsprechende Rohrleitungen zu entsprechend anderen Anlagenteilen geführt sind.

Bei der modularen Unterbringung in Containern ist jedoch von Nachteil, dass beim Auslaufen von Betriebsmitteln, wie Ölen aber auch anderen Flüssigkeiten handelsübliche Container nicht dicht sind. Dementsprechend müssen derartige Container durch das Einschweißen zusätzlicher Bleche aufgerüstet werden, was besonders teuer ist. Ferner überträgt sich die Schwingung der Verbrennungsmotoren ohne besondere Maßnahmen auf den Container, der üblicherweise aus Stahlblech besteht, welches Schwingungsprobleme nach sich zieht.

Durch den Aufbau der Container in bekannter Weise aus Stahlblechen ergibt sich zudem eine Schallschutzproblematik, da die Blockheizkraftwerke in erheblicher Weise Lärm erzeugen.

Aus der US 5,656,491 A geht eine Anlage aus mobilen Modulen zur Entwicklung und Herstellung von biotechnologischen Produkten im Pilotmaßstab hervor. Die Anlage besteht aus wenigsten zwei Modulen die miteinander verbindbar und zusammen integrierbar sind. Jedes der mobilen Module besteht aus einem mobilen Container, wobei zumindest einer der Container eine aseptische Umgebung bildet. Die Module mit aseptischer Umgebung können Tanks für Versorgungsstoffe, Säure-, Lauge-, und Pufferlösungen, Bioreaktoren und andere Geräte zum Kultivieren von Mikroorganismen und anderen, gegebenenfalls modifizierten, wie etwa genetisch veränderten biologischen Materialien, Zentrifugen, Ultrafiltrations- und Homogenisationsapparaten, Chromatographiesäulen, Gefriertrockner, Autoklaven und anderen Geräten, die üblicherweise in biotechnologischen Verfahren verwendet werden enthalten. Die anderen Module können Geräte enthalten, wie Klimaanlagen, Generatoren, Produktionseinheiten für Kühlwasser, Erhitzer für Reindampf und Industriedampf, Wasserenthärter, Umkehrosmoseeinheiten, Luftkompressoren und ähnliches. Die einzelnen Module können über zusätzliche Verbindungsmodule verbunden werden, die die Module für das Bedienungspersonal zugänglich machen und ebenfalls aseptisch sind. Da die Art und Anzahl der benötigten Module variiert werden kann soll es möglich sein, eine Anlage zu schaffen, die genau den Bedürfnissen des Benutzers entspricht und eine hohe Flexibilität aufweist. Zudem wird auf diese Weise ein schnelles aufund abbauen der Anlage sowie eine Verwendung zu einem anderen Zweck ermöglicht.

In der DE 199 58 142 A1 ist eine mobile Biogasanlage beschrieben. Die Biogasanlage soll durch einen modularen Aufbau kostengünstig zu transportieren und die Fermenterkapazität bedarfsgerecht angepasst werden können. Die Anlagenteile sollen mit konventionellen, für den Transport von Standardcontainem ausgelegten LKW-Zügen befördert werden können. Die Anlage besteht aus einem Fermenter und einem Energieteil die in mindestens zwei voneinander getrennten Standardcontainem bzw. Standardcontainerrahmen untergebracht sind. Das System kann durch den modularen Aufbau während des Betriebes von einem Biogasnutzungssystem (z.B. Verstromung/Wärmeerzeugung im Blockheizkraftwerk) auf ein anderes (zum Beispiel katalytische Verbrennung, bzw. Abfüllung des Biogases) umgestellt werden.

Aus der WO 94/19120 A geht eine Vorrichtung zur Förderung von Biogas aus Mülldeponien hervor. Das geförderte Biogas wird innerhalb der Anlage einem Boiler oder einem motorbetriebenen Generator zugeführt, der das geförderte Biogas in Strom umwandelt. Das System beinhaltet ein Modul, welches das Gas dem Boiler oder dem motorbetriebenen Gasgenerator zuführt. Das Modul befindet sich in einem Standardcontainer, der in einem Kontrollraum und einem Pumpenraum unterteilt ist. Der Kontrollraum und der Pumpenraum sind gasdicht durch eine Wand voneinander getrennt. Im Pumpenraum befinden sich die Steuer- und Messeinrichtungen der Anlage.

In der EP 0 264 567 A2 ist ein Bioreaktor beschrieben. Der Bioreaktor besteht aus einem Reaktortank und mehreren darin getrennt voneinander angeordneten Reaktormodulen. Diese Reaktormodule werden von einer in den Reaktortank führenden Rohwasserleitung gemeinsam gespeist. Der Reaktortank dient zugleich als Vorratsbehälter für das Rohwasser. Dadurch ist kein separater Behälter für die Rohwasserspeicherung mehr nötig und es ergibt sich die Möglichkeit mit dem großen Flüssigkeitsvolumen eine einfache Temperaturregelung zu realisieren. Der Reaktortank wirkt als Wärmespeicher, der eine viel kleinere Wärme abgebende Oberfläche als die einzelnen Module hat. Aus diesem Grund kann mit einem Wärmetauscher und einer relativ kleinen Pumpe leicht die für den biologischen Vorgang gewünschte Temperatur erreicht und eingehalten werden.

In der DIN EN 13978-1 sind Betonfertiggaragen definiert. Hierin sind umfassende Anforderungen an die Baustoffe, an die Herstellung, an die Widerstandsfähigkeit gegen mechanische Einwirkungen, an den Feuerwiderstand und das Brandverhalten, an die schallschutztechnischen Eigenschaften, an die wärmeschutztechnischen Eigenschaften und an die Dauerhaftigkeit angegeben.

Aufgabe der Erfindung ist es, eine Biogasanlage in modularem Aufbau zu Verfügung zu stellen, welche eine hohe Flexibilität besitzt, wobei die einzelnen Module dicht sind, ein verbessertes Schallschutzverhalten und Schwingungsverhalten besitzen und zudem deutlich günstiger als bekannte modulare Biogasanlagen sind.

Die Aufgabe wird mit einer Biogasanlage mit den Merkmalen des Anspruchs 1 und durch ein Modul mit den Merkmalen des Anspruchs 7 gelöst.

Vorteilhafte Weiterbildungen werden in Unteransprüchen gekennzeichnet.

Erfindungsgemäß werden als die Module aufnehmende bzw. Module ausbildende Gehäuse Fertiggaragen verwendet. Diese Fertiggaragen lassen sich in einfacher Weise mit den jeweiligen technischen Einheiten bestücken und transportieren. Durch den Aufbau aus mineralischen Baustoffen und insbesondere Beton sind die Wände besonders schalldicht, da sie schwer sind. Ferner sind die verwendeten Fertiggaragen, da sie für die Aufnahme von Kraftmaschinen von vornherein ausgebildet sind, auch dicht gegenüber austretenden Betriebsflüssigkeiten, wobei die verwendeten Baustoffe auch bezüglich auftretender Schwingungen besonders unproblematisch sind.

Erfindungsgemäß ist neben dem Fermenter beispielsweise ein Basismodul vorhanden, wobei dieses Basismodul als Fertiggarage ausgebildet ist, welche die Pumpentechnik, die Belüftungstechnik, die Elektrotechnik und die Steuerung aufnimmt. Zudem ist erfindungsgemäß zumindest ein weiteres Blockheizkraftwerkmodul vorhanden, bei dem in zumindest einer Fertiggarage ein Motor, ein Generator, die entsprechende Schaltungstechnik, ein Wärmetauscher, eine Rückkühltechnik und die Gasaufbereitung vorhanden sind. Diese Module können an sich bekannterweise die ohnehin an Fertiggaragen vorhandenen Zugänge, wie Garagentor und/oder Seitentür besitzen. Ferner besitzen die entsprechenden Module erfindungsgemäß an den Außenwandbereichen oder in den Deckenbereichen Anschlussschnittstellen zum Anschließen von Rohrleitungen oder elektrischen Ver- oder Entsorgungsleitungen. Vorzugsweise werden die Anschlussschnittstellen so standardisiert, dass eine Standardschnittstelle eines Steuerungsmoduls beispielsweise für eine Standardanschlussgeometrie eines Blockheizkraftwerkmoduls vorhanden ist.

Vorzugsweise besitzt beispielsweise das Basismodul, welches die Steuerungstechnik und Pumpentechnik aufnimmt zudem leere Schnittstellplätze oder bereits vorhandene Schnittstellen für weitere Blockheizkraftwerkmodule. So ist es insbesondere möglich beispielsweise eine Mehrzahl von Blockheizkraftwerkmodulen an ein gemeinsames Basismodul anzuschließen.

Wird eine entsprechend aufgebaute Biogasanlage schrittweise vergrößert, ist es somit möglich, einen Fermenter mit einem Basismodul und zunächst beispielsweise einem Blockheizkraftwerkmodul zu verbinden und bei steigender Biogaserzeugung beispielsweise bis zu 4 Blockheizkraftwerkmodule an das Basismodul anzuschließen. Sind die Kapazitäten des Basismoduls erschöpft, kann dann beispielsweise ein weiteres Basismodul hinzugestellt werden. Vorzugsweise besitzen die Basismodule deshalb auch Schnittstellen für die Vernetzung bzw. Verschaltung mehrerer Basismodule untereinander.

Bei der Erfindung ist von Vorteil, dass eine Biogasanlage geschaffen wird, die modulartig flexibel aufbaubar ist, wobei die einzelnen Module in Fertiggaragen untergebracht sind, welche eine gute Dichtheit, einen verbesserten Schallschutz, verminderte Schwingungsprobleme und geringere Kosten verursachen.

Die Erfindung wird anhand einer Zeichnung beispielhaft erläutert. Es zeigen dabei:
- Figur 1:: ein Biogasanlagen-Basismodul in einer seitlichen teilgeschnittenen Ansicht;
- Figur 2:: das Modul nach Figur 1 in einer schematischen Draufsicht;
- Figur 3:: das Modul nach Figur 1 in einer rückseitigen teilgeschnittenen Ansicht;

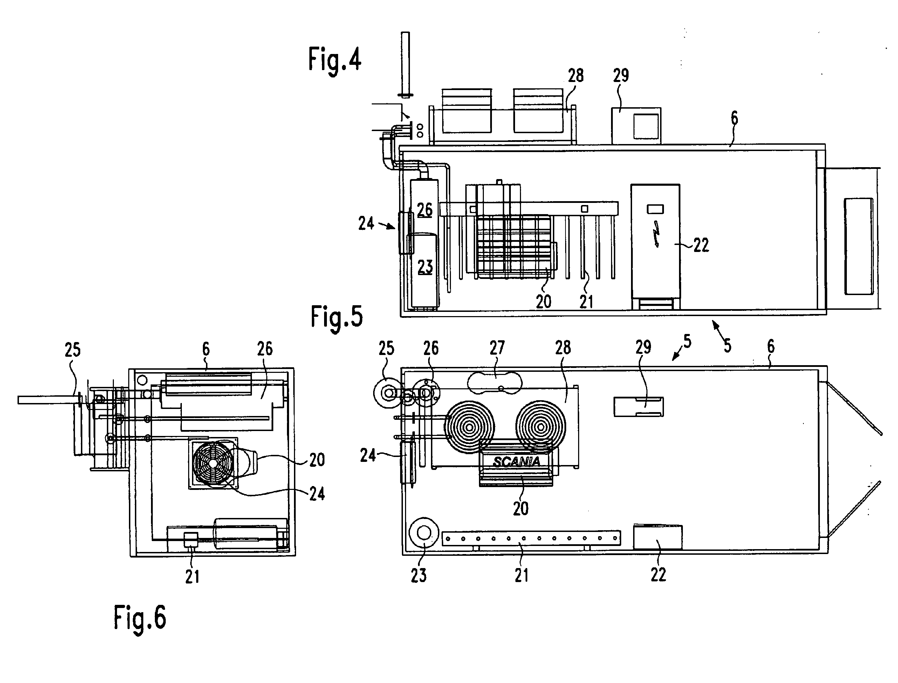

- Figur 4:: ein Biogasanlagen-Blockheizwerkmodul in einer teilgeschnittenen seitlichen Ansicht;
- Figur 5:: das Modul nach Figur 4 in einer teilgeschnittenen Draufsicht von oben;
- Figur 6:: das Modul nach Figur 4 in einer teilgeschnittenen rückseitigen Ansicht;
- Figur 7:: eine perspektivische Ansicht einer erfindungsgemäßen Biogasanlage;
- Figur 8:: eine Biogasanlage nach Figur 7 in einer erweiterten und vergrößerten Ausführungsform.

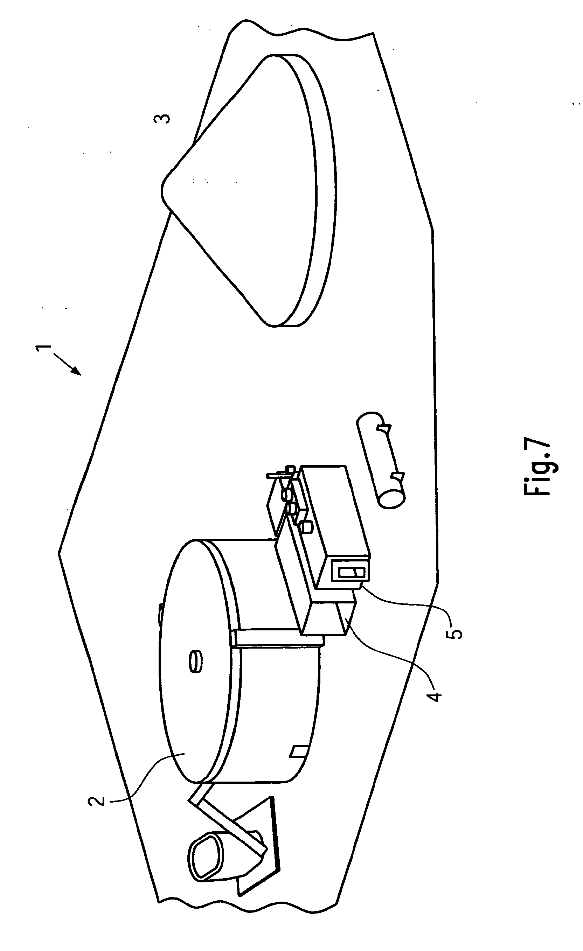

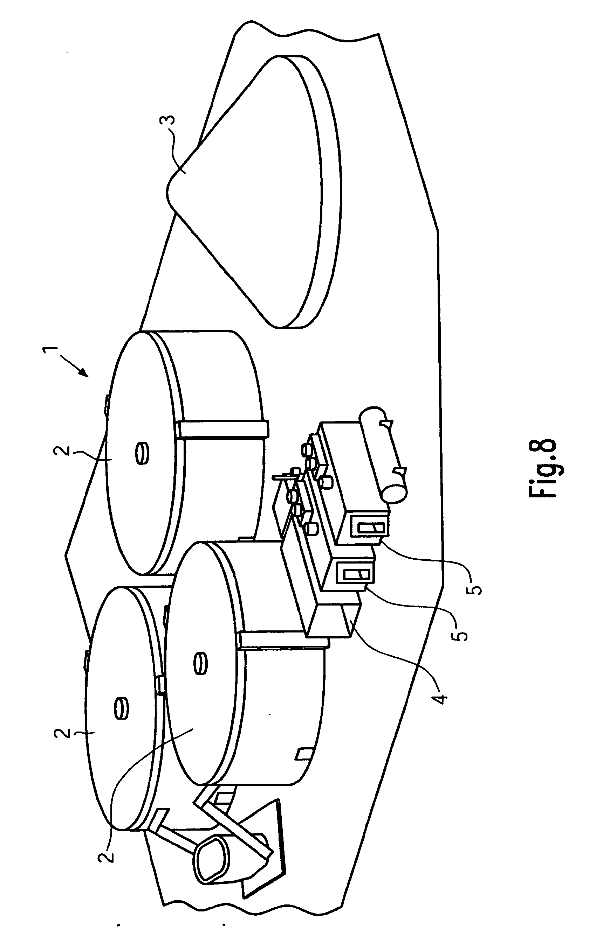

Eine erfindungsgemäße Biogasanlage 1 (Figuren 7, 8) besitzt zumindest einen Fermenter 2, ein Abraumlager für fermentiertes Gut 3 sowie zumindest ein Basismodul 4 und ein Blockheizkraftwerkmodul 5.

Das Basismodul 4 und das Blockheizkraftwerkmodul 5 sind jeweils in einer Fertiggarage 6 ausgebildet. Eine Fertiggarage im Sinne der vorliegenden Erfindung ist ein quaderförmiger standardisierter Baukörper mit zumindest einer Bodenplatte und mehreren Seitenwandungen aus mineralischen Baustoffen, insbesondere Beton, wobei vorzugsweise auch eine Deckenplatte aus diesen mineralischen Baustoff ausgebildet ist. Die Bodenplatte, Seitenwandungen und Deckenplatte bilden einen einstückigen Körper. Die Fertiggarage weist eine oder mehrere Öffnungen im Bereich der Seitenwandungen auf, die mit Türen und/oder Toren zum Schließen der Öffnungen versehen sind.

Derartige Fertiggaragen werden in großen Stückzahlen gefertigt, weshalb sie günstig sind und gleichzeitig hohen Ansprüchen bezüglich Schallschutz, Schwingungsdämpfung und Dichtigkeit genügen.

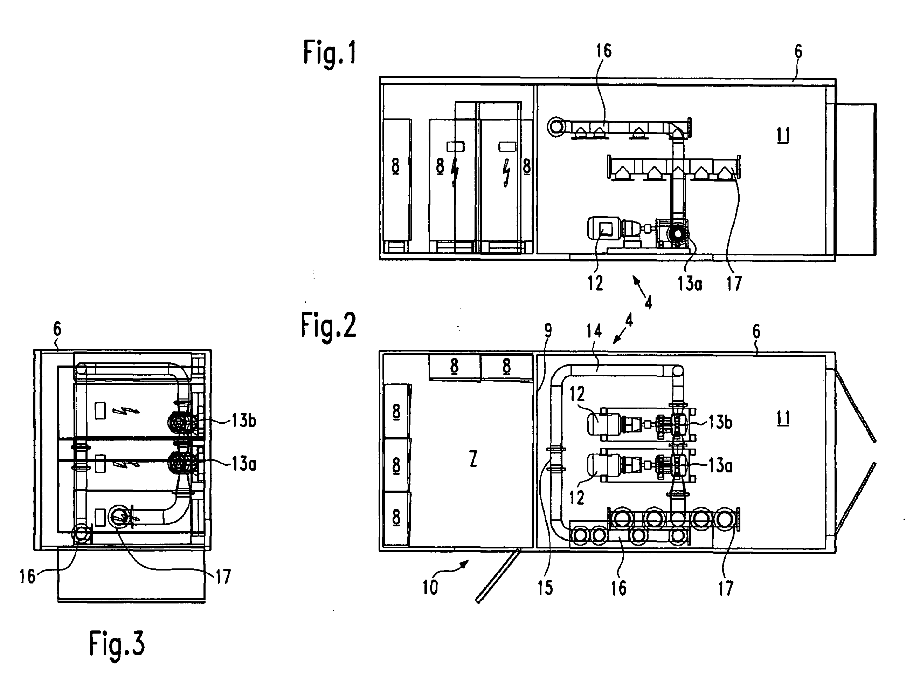

Das Basismodul 4 umfasst wir bereits ausgeführt eine Fertiggarage 6, die mit einer Wandung 9 unterteilt ist (Fig. 1 bis 3). Hierdurch wird ein Schaltraum 7 und ein Pumpenraum 11 gebildet. Der Schaltraum 7 umfasst Schaltschränke 8 für die gesamte Anlage sowie insbesondere Industrie-PCs zum Betreiben der Anlage. Die Trennwandung 9 trennt den Schaltraum 7 und den Pumpenraum 11 hermetisch voneinander ab.

In den Schaltraum 7 führt ein beispielsweise seitlich am Garagenkorpus angeordneter Eingang 10.

Der Pumpenraum 11 umfasst zwei Elektromotoren 12, die beispielsweise axial nebeneinander angeordnet sind. Hierbei wirkt einer der Motoren 12 auf einen Zerhacker 13a zum Aufschluss des vergorenen Substrates und der danebenliegende Motor 12 auf eine Drehkolbenpumpe 13b, wobei der Zerhacker 13a und die Drehkolbenpumpe 13b fluidisch leitend miteinander verbunden sind.

Von der Drehkolbenpumpe 13b verläuft eine Verrohrung 14 zu einem Verteilerbalken 16 mit pneumatischen Schiebern der Druckseite mit entsprechenden Anschlüssen zur Anbindung weiterer Druckleitungen zum Äußeren des Basismoduls 4. Dementsprechend ist dem Zerhacker 13a ein Verteilerbalken 17 mit pneumatischen Schiebern auf der Saugseite vorgeordnet, ebenfalls mit entsprechenden Anschlüssen zum Anbinden aller Saugleitungen. Zudem kann in der Verrohrung 14 eine Durchflussmengenmessvorrichtung 15 vorhanden sein. Der Verteilerbalken 17 auf der Saugseite ist mit Leitungen verbunden, die zum Fermenter, zu einem Nachgärbehälter (nicht dargestellt) und zu einer Vorgruppe führen, von welcher aus die Fermenter beschickt wird. Der Verteilerbalken 16 auf der Druckseite ist mit Leitungen verbunden, die zum Fermenter, zur Vorgruppe, zum Nachgärbehälter und zum Abraumlager führen. Daher ist die Pumpe universell einsetzbar, um das Gut von einem zu einem anderen Behälter zu befördern, oder auch im Fermenter umzuwälzen, indem sie das Gut aus dem Fermenter abzieht und wieder dem Fermenter zuführt.

Zudem können im Bereich des Pumpenraums 11 Bodenaussparungen oder Vorbereitungen für Bodenaussparungen vorhanden sein, um eine frostsichere Rohrleitungsführung durch den Boden in das Erdreich zu ermöglichen.

Ferner können vorzugsweise in dem Pumpenraum 11 eine Kompressoranlage (nicht gezeigt) zur Versorgung der pneumatischen Schieber mit Druckluft und gegebenenfalls ein Seitenkanalverdichter zur Erzeugung des Luftstroms zur biologischen Entschwefelung (ebenfalls nicht dargestellt) vorhanden sein.

Das Blockheizkraftwerkmodul 5 ist ebenfalls in einer Fertiggarage 6 untergebracht. Das Blockheizkraftwerkmodul 5 umfasst ein Motormodul 20 mit einem an das Motormodul angeschlossenen Generator (nicht gezeigt).

Zudem ist dem Motormodul 20 ein Ausdehnungsgefäß 23 für den Motor 20 vorgesehen, sowie ein Wasserkühler 29, der auf dem Dach der Garage 6 angeordnet ist. Zusätzlich umfasst das Blockheizkraftwerkmodul 5 einen Notkühler 28, der ebenfalls auf dem Dach der Garage 6 angeordnet ist.

Um die entstehende Wärme effektiv nutzen zu können, ist zudem ein Wärmeverteiler mit Umwälzpumpe 21 zur Nutzung der Abwärme im Blockheizkraftwerk in dem Modul 5 angeordnet, wobei zusätzlich ein Abgaswärmetauscher 26 vorhanden ist, durch den die Abgase geleitet werden, bevor sie in ein entsprechendes Abgasrohr 25 mit Schalldämpfer auf dem Garagendach eingeleitet werden.

Zudem ist ein Ventilator 24 zur Entlüftung der Garage vorgesehen, wobei vorzugsweise der Ventilator 24 so angeordnet ist, dass der Luftstrom über das Motormodul 20 geführt ist.

Zudem ist eine Biogasaufbereitung 27 vorhanden, die beispielsweise aus einem Aktivkohlefilter und einer Biogasentfeuchtung besteht, um das Biogas vor der Einleitung in das Blockheizkraftwerk entsprechend aufzubereiten. Zur entsprechenden Schaltung und Steuerung des Blockheizkraftwerkes ist zudem ein Schaltstrang 22 vorgesehen.

Eine Biogasanlage 1 umfasst in einer Grundausbaustufe beispielsweise einen Fermenter 2 und ein Abraumlager 3 für fermentiertes Gut sowie ein Basismodul 4 und ein Blockheizkraftwerkmodul 5. Wird eine derartige Anlage erweitert (Figur 12) werden beispielsweise zwei weitere Fermenter hinzugebaut, welche über die entsprechenden Anschlüsse 16, 17 mit dem Basismodul 4 verbunden werden. Um die größer gewordene Gasmenge nutzen zu können, insbesondere ohne einen Zwischenspeicher anlegen zu müssen, wird dementsprechend ein weiteres Blockheizkraftwerkmodul 5 hinzugestellt und mit den entsprechenden Rohrleitungen 16, 17 bzw. Anschlüssen 16, 17 verbunden.

Das Basismodul 4 ist mit dem bzw. den Blockheizkraftwerkmodulen 5 lediglich über eine elektrische Datenleitung und eine Stromversorgungsleitung verbunden. Es bestehen keine Rohrleitungen, die direkt von einem Modul zu einem weiteren Modul führen. Der Gasanschluß der Blockheizkraftwerkmodule 5 erfolgt mittels einer Rohrleitung, die vom Fermenter durch die Bodenplatte des Blockheizkraftmoduls 5 geführt wird.

Aus Sicherheitsgründen ist es wichtig, dass der Pumpenraum 11 separat vom Blockheizkraftwerkmodul 5 ausgeführt ist.

Bei der Erfindung ist wesentlich, dass die Module 4, 5 in Fertiggaragen 6 untergebracht sind, welche eine einfache und sichere, dichte, schallgeschützte und kostengünstige sowie gegen Vibrationen und Schwingungen unempfindliche Anlage erlauben.

## Patentansprüche

1. Biogasanlage mit zumindest einem Fermenter sowie Modulen, welche technische Teile der Anlage aufnehmen, insbesondere die Steuerungs- und Regelungstechnik, die Pumpentechnik sowie zumindest ein Blockheizkraftwerk, **dadurch gekennzeichnet, dass** die Module (4, 5) als Umhausung bzw. Gehäuse und die technischen Einheiten aufnehmende Behältnisse Fertiggaragen (6) besitzen, wobei die Fertiggaragen (6) aus mineralischen Baustoffen ausgebildet sind, welche für die Aufnahme der Module (4, 5) entsprechend ausgebildet sind.

2. Biogasanlage nach Anspruch 1,
**dadurch gekennzeichnet,**
**dass** ein Modul (4) ein Basismodul ist, welches die Pumpentechnik und/oder die Belüftungstechnik und/oder die Elektrotechnik und/oder die Steuerung und Regelung aufnimmt.

3. Biogasanlage nach Anspruch 1 oder 2,
**dadurch gekennzeichnet,**
**dass** ein Modul (5) als Blockheizkraftwerkmodul ausgebildet ist und zumindest einen Verbrennungsmotor (20) und/oder einen Generator und/oder die Schaltungstechnik hierfür und/oder einen Wärmetauscher und/oder eine Rückkühlvorrichtung und/oder eine Gasaufbereitung umfasst.

4. Biogasanlage nach einem der vorhergehenden Ansprüche,
**dadurch gekennzeichnet,**
**dass** die Module (4, 5) Anschlussschnittstellen umfassen zum Anschließen mit anderen Modulen (4, 5) und/oder Anlagen teilen.

5. Biogasanlage nach Anspruch 4,
**dadurch gekennzeichnet,**
**dass** die Anschlussschnittstellen Anschlüsse für die Stromversorgung und für die Datenleitungen der Schaltungstechnik umfassen.

6. Biogasanlage nach Anspruch 4 oder 5,
**dadurch gekennzeichnet,**
**dass** die Anschlussschnittstellen jeweils für den Anschluss an einem weiteren Modul (4, 5) oder eines weiteren Moduls (4, 5) oder eines weiteren außerhalb eines Moduls liegenden Anlagenteils standardisiert sind.

7. Modul für eine Biogasanlage, insbesondere für eine Biogasanlage nach einem der Ansprüche 1 bis 6, das Teile der Biogasanlage (1), insbesondere die Steuerungs- und Regelungstechnik, die Pumpentechnik (21) und/oder ein Blockheizkraftwerk umfasst, die in einem Gehäuse angeordnet sind,
**dadurch gekennzeichnet,**
**dass** das Gehäuse eine Fertiggarage (6) ist, die aus mineralischem Baustoff ausgebildet ist.

8. Modul nach Anspruch 7,
**dadurch gekennzeichnet,**
**dass** das Modul (5) als Blockheizkraftwerkmodul ausgebildet ist.

9. Modul nach Anspruch 7,
**dadurch gekennzeichnet,**
**dass** das Modul (4) als Basismodul ausgebildet ist, welches die Pumpentechnik und/oder Belüftungstechnik und/oder Elektrotechnik und/oder die Steuerung und Regelung umfasst.

10. Modul nach einem der Ansprüche 7 bis 9,
**dadurch gekennzeichnet,**
**dass** die Fertiggarage (6) ein quaderförmiger Baukörper mit zumindest einer Bodenplatte und mehreren Seitenwandungen aus mineralischen Baustoffen, insbesondere Beton, ist, wobei vorzugsweise auch eine Deckenplatte aus diesem mineralischen Baustoff ausgebildet ist.

11. Modul nach Anspruch 10,
**dadurch gekennzeichnet,**
**dass** die Bodenplatte, die Seitenwandungen und die Deckenplatte einen einstückigen Körper bilden.

12. Modul nach einem der Ansprüche 7 bis 11,
**dadurch gekennzeichnet,**
**dass** die Fertiggarage (6) eine oder mehrere Öffnungen im Bereich der Seitenwandungen aufweist, die mit Türen und/oder Toren zum Verschließen der Öffnung versehen sind.

## Claims

1. Biogas plant with at least one fermenter and with modules which accommodate technical parts of the plant, in particular the process control unit, the pump system and at least one combined heat and power plant, **characterised in that** the modules (4, 5) have prefabricated garages (6) as housing or casing and as containers accommodating the technical units, wherein the prefabricated garages (6) are made of mineral construction materials which are suitably designed to accommodate the modules (4, 5).

2. Biogas plant according to claim 1,
**characterised in that**
one module (4) is a basic module which accommodates the pump system and/or the ventilation system and/or the electrical engineering equipment and/or the process control unit.

3. Biogas plant according to claim 1 or 2,
**characterised in that**
one module (5) is in the form of a combined heat and power plant module comprising at least one internal combustion engine (20), and/or a generator and/or the circuit logic for the latter and/or a heat exchanger and/or a re-cooling unit and/or a gas processing unit.

4. Biogas plant according to any of the preceding claims,
**characterised in that**
the modules (4, 5) include connection interfaces for connection with other modules (4, 5) and/or plant components.

5. Biogas plant according to claim 4,
**characterised in that**
the connection interfaces contain connections for the power supply and for the data lines of the circuit logic system.

6. Biogas plant according to claim 4 or 5,
**characterised in that**
each of the connection interfaces is standardised for connection to another module (4, 5) or of another module (4, 5) or of another plant component situated outside a module.

7. Module for a biogas plant, in particular for a biogas plant according to any of claims 1 to 6, and containing parts of the biogas plant (1), in particular the process control unit, the pump system (21) and/or a combined heat and power plant, mounted in a housing,
**characterised in that**
the housing is a prefabricated garage (6) made of mineral construction material.

8. Module according to claim 7
**characterised in that**
the module (5) is in the form of a combined heat and power plant.

9. Module according to claim 7
**characterised in that**
the module (4) is designed as a basic module which contains the pump system and/or the ventilation system and/or the electrical engineering equipment and/or the process control unit.

10. Module according to any of claims 7 to 9
**characterised in that**
the prefabricated garage (6) is a cuboidal building with at least one floor panel and several side walls of mineral construction materials, in particular concrete, wherein preferably a ceiling is also made of this mineral construction materials.

11. Module according to claim 10
**characterised in that**
the floor panel, the side walls and the ceiling form an integral body.

12. Module according to any of claims 7 to 11
**characterised in that**
the prefabricated garage (6) has in the area of the side walls one or more openings provided with doors and/or gates to close the opening.

## Revendications

1. Installation de biogaz comprenant au moins un fermenteur ainsi que des modules qui reçoivent les parties techniques de l'installation, en particulier les organes techniques de commande et de régulation, les organes techniques de pompage, ainsi qu'au moins une centrale de chauffage à énergie totale, **caractérisée en ce que** les modules (4, 5) possèdent, à titre de carter ou de boîtier et de réceptacles recevant les unités techniques, des garages prêts à poser (6), lesdits garages prêts à poser (6) étant réalisés en matériaux de construction minéraux, qui sont réalisés de façon correspondante pour la réception des modules (4, 5).

2. Installation de biogaz selon la revendication 1,
**caractérisée en ce qu'**un module (4) est un module de base, qui reçoit les organes techniques de pompage et/ou les organes techniques de ventilation et/ou les organes techniques électriques et/ou la commande et la régulation.

3. Installation de biogaz selon la revendication 1 ou 2,
**caractérisée en ce qu'**un module (5) est réalisé à titre de module de centrale de chauffage à énergie totale, et comprend au moins un moteur à combustion interne (20) et/ou un générateur et/ou les organes techniques de commutation associés et/ou un échangeur de chaleur et/ou un dispositif de refroidissement et/ou une unité de traitement de gaz.

4. Installation de biogaz selon l'une des revendications précédentes,
**caractérisée en ce que** les modules (4, 5) comprennent des interfaces de raccordement pour le raccordement à d'autres modules (4, 5) et/ou d'autres parties de l'installation.

5. Installation de biogaz selon la revendication 4,
**caractérisée en ce que** les interfaces de raccordement comprennent des raccordements pour l'alimentation électrique et pour les lignes véhiculant les données de l'unité technique de commutation.

6. Installation de biogaz selon la revendication 4 ou 5,
**caractérisée en ce que** les interfaces de raccordement sont respectivement standardisées pour le raccordement à un autre module (4, 5) ou d'un autre module (4, 5), ou d'une autre partie de l'installation située à l'extérieur d'un module.

7. Module pour une installation de biogaz, en particulier pour une installation de biogaz selon l'une des revendications 1 à 6, qui comprend des parties de l'installation de biogaz (1), en particulier les organes techniques de commande et de régulation, les organes techniques de pompage (21) et/ou une centrale de chauffage à énergie totale, qui sont agencés dans un boîtier,
**caractérisé en ce que** le boîtier est un garage prêt à poser (6), qui est réalisé à partir de matériaux de construction minéraux.

8. Module selon la revendication 7,
**caractérisé en ce que** le module (5) est réalisé sous forme de module de centrale de chauffage à énergie totale.

9. Module selon la revendication 7,
**caractérisé en ce que** le module (4) est réalisé sous forme de module de base, qui comprend les organes techniques de pompage et/ou les organes techniques de ventilation et/ou les organes techniques électriques et/ou la commande et la régulation.

10. Module selon l'une des revendications 7 à 9,
**caractérisé en ce que** le garage prêt à poser (6) et un corps structurel de forme parallélépipédique avec au moins une plaque de plancher et plusieurs parois latérales en matériaux de construction minéraux, en particulier en béton, et dans lequel une plaque de toiture est également réalisée à partir de ce matériau de construction minéral.

11. Module selon la revendication 10,
**caractérisé en ce que** la plaque de plancher, les parois latérales et la plaque de toiture forment un corps d'un seul tenant.

12. Module selon l'une des revendications 7 à 11,
**caractérisé en ce que** le garage prêt à poser (6) comporte une ou plusieurs ouvertures dans la région des parois latérales, qui sont pourvues de portes et/ou de portails pour la fermeture de l'ouverture.
